# EUROPÄISCHE PATENTSCHRIFT

(11) **EP 0 547 499 B1**
(45) Veröffentlichungstag und Bekanntmachung des Hinweises auf die Patenterteilung: **27.03.1996**
(21) Anmeldenummer: 92121029.0
(22) Anmeldetag: 10.12.1992
(51) Int. Cl.: C07C 211/38, C07C 209/26

(54) **Verfahren zur Herstellung von 8-N,N-Dialkylaminotricyclo-(5.2.1.0 2,6)decan**
Procedure for the preparation of 8-N,N-dialkylaminotricyclo(5.2.1.02,6)decane
Procédé de préparation de 8-N,N-dialcoyl-aminotricyclo(5.2.1.02,6)décane

(30) Priorität: 19.12.1991 DE 4141962
(43) Veröffentlichungstag der Anmeldung: 23.06.1993
(73) Patentinhaber: HOECHST AKTIENGESELLSCHAFT, D-65926 Frankfurt am Main (DE)
(72) Erfinder: Kniep, Claus, W-4200 Oberhausen 1 (DE); Kampmann, Detlef, Dr. Dipl.-Chem., W-4630 Bochum (DE); Weber, Jürgen, Dr. Dipl.-Chem-, W-4200 Oberhausen 11 (DE)

(56) Entgegenhaltungen:
- WO-A-90/04567
- JOURNAL OF THE AMERICAN CHEMICAL SOCIETY, Bd. 81, Nr. 3, 12. Februar 1959, Washington, DC, US, Seiten 655 - 658; P. WILDER et al: "1,2-Dihydro-endo-dicyclopentadiene"

## Beschreibung

Die vorliegende Erfindung betrifft ein Verfahren zur Herstellung von 8-N,N-Dialkylaminotricyclo [5.2.1.0^{2,6}] decan. Durch Umsetzung von 8-N,N-Dialkylaminotricyclo [5.2.1.0^{2,6}] decan mittels eines Alkylhalogenids lassen sich die entsprechenden quaternären Ammoniumsalze des Tricyclo [5.2.1.0^{2,6}] decans herstellen. Diese quarternären Ammoniumsalze des Tricyclo [5.2.1.0^{2,6}] decans werden beispielsweise für die Herstellung von Zeolithen benötigt.

Eine mehrstufige Synthese von 9-Dimethylaminotetrahydrodicyclopentadien (8-N,N-Dimethylaminotricyclo [5.2.1.0^{2,6}] decan) ist von P. Wilder, Jr., Ch. F. Culberson und G. T. Youngblood in J. Am. Chem. Soc. 81, 655 bis 658 (1959) beschrieben. Ausgehend von einem Keton (Tricyclo [5.2.1.0^{2,6}] dec-3-en-8(9)-on wird durch Umsetzung mit Hydroxylamin das entsprechende Oxim hergestellt. Das Oxim wird in zwei separaten Schritten (zunächst mittels PtO₂ und H₂ und anschließend mit Natrium) zu dem entsprechendem Amin (8-Aminotricyclo [5.2.1.0^{2,6}] decan) reduziert. Das Amin läßt sich mit einer Mischung aus wäßrigem Formaldehyd und Ameisensäure zu dem entsprechenden (9)-Dimethylaminotetrahydrodicyclopentadien (8-N,N-Dimethylaminotricyclo [5.2.1.0^{2,6}] decan) umsetzen. Die Ausbeute in der Stufe der Methylierung beträgt lediglich 48 % bezogen auf eingesetztes primäres Amin, die Ausbeute bezogen auf das gesamte Herstellungsverfahren liegt jedoch entsprechend niedriger.

Die WO 90/04567 beschreibt ein Verfahren zur Herstellung von N,N-Dialkyl-8-aminotricyclo [5.2.1.0] decan (8-N,N-Dialkylaminotricyclo [5.2.1.0^{2,6}] decan), das von 8-Ketotricyclo [5.2.1.0] decan (Tricyclo [5.2.1.0^{2,6}] decan-8-on) ausgeht. Hierbei setzt man das Keton mit einem Dialkylformamid in Anwesenheit von Ameisensäure bei Temperaturen von 160 bis 195°C um. Die Reaktionszeit beträgt 10 bis 50 Stunden. Bei der Umsetzung entsteht CO₂, das zur Vermeidung eines unerwünschten Druckanstieges laufend aus der Reaktion entfernt werden muß.

Die vorstehend beschriebenen Verfahren sind nicht nur technisch recht aufwendig, sondern erfordern auch einen hohen Zeitaufwand.

Daher besteht die Aufgabe, ein Verfahren zu entwickeln, das sowohl einfach auszuführen ist, als auch das gewünschte 8-N,N-Dialkylaminotricyclo [5.2.1.0^{2,6}] decan in hoher Ausbeute zugänglich macht und zudem den Zeitaufwand für die Durchführung der Umsetzung verringert.

Diese Aufgaben werden gelöst durch ein Verfahren zur Herstellung von 8-N,N-Dialkylaminotricyclo [5.2.1.0^{2,6}] decan. Es ist dadurch gekennzeichnet, daß man Tricyclo [5.2.1.0^{2,6}] decan-8-on mit einem Amin der Formel HNR₁R₂, worin R₁ und R₂ für einen Alkylrest mit 1 bis 6 Kohlenstoffatomen stehen, in Gegenwart eines Platin enthaltenden Trägerkatalysators mit Wasserstoff bei erhöhtem Druck und erhöhter Temperatur umsetzt.

Das für die Umsetzung benötigte Tricyclo [5.2.1.0^{2,6}] decan-8-on läßt sich in technischem Maßstab ausgehend von Dicyclopentadien gewinnen. Durch Wasseranlagerung entsteht in Anwesenheit von sauren Katalysatoren (H₂SO₄ oder Kationenaustauscher) 8(9)-Hydroxytricyclo [5.2.1.0^{2,6}] dec-3-en, ein ungesättigter Alkohol, der mit Hilfe eines Nickel enthaltenden Katalysators bei erhöhter Temperatur in das Tricyclo-[5.2.1.0^{2,6}] decan-8-on überführt werden kann. Bei dieser Umsetzung handelt es sich aller Wahrscheinlichkeit nach um eine intramolekulare Wasserstoffumlagerung, wobei - entsprechend der nachfolgenden Reaktionsgleichung - die in 8- bzw. 9-Position befindliche Hydroxylgruppe unter Abspaltung von Wasserstoff in eine Keto-gruppe umgewandelt wird und der freigesetzte Wasserstoff an die in 3-Stellung angeordnete Kohlenstoff-Kohlenstoff-Doppelbindung angelagert wird.

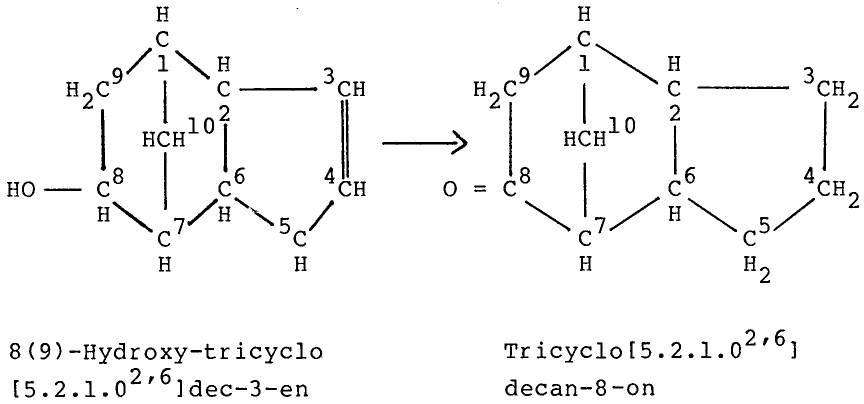

Auf diese Weise wandelt sich der ungesättigte Alkohol in das Tricyclo [5.2.1.0^{2,6}] decan-8-on um.

Folgend der Gleichung: 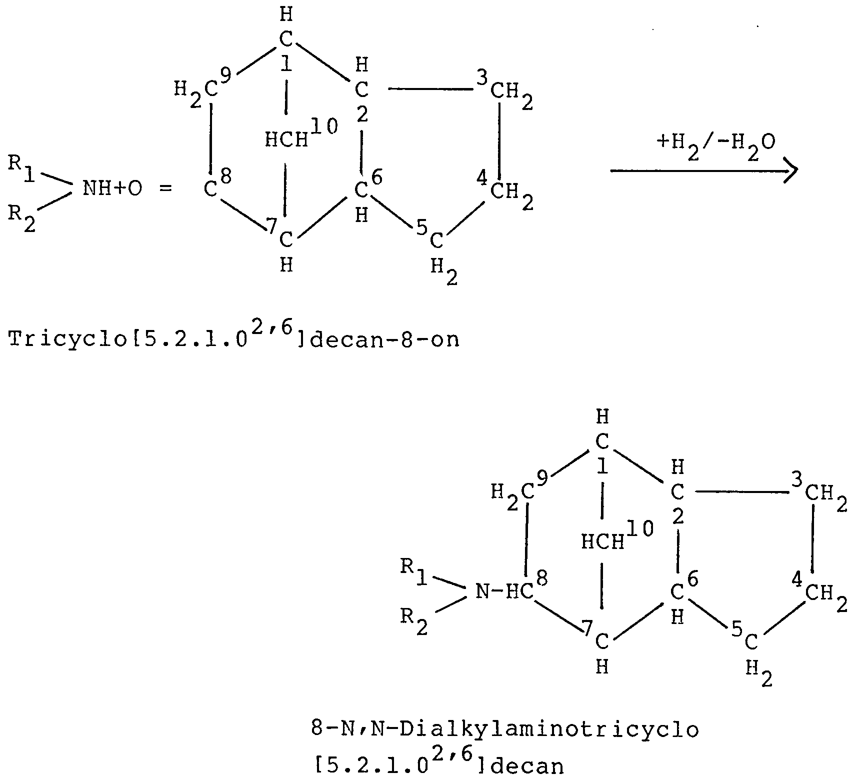
entsteht aus Tricyclo [5.2.1.0^{2,6}] decan-8-on und dem Amin der Formel R₁R₂NH das gewünschte 8-N,N-Dialkylaminotricyclo [5.2.1.0^{2,6}] decan.

Die Ausgangsstoffe Tricyclo [5.2.1.0^{2,6}] decan-8-on und das Amin der Formel R₁R₂NH können in äquivalenten Mengen eingesetzt werden. Hiervon abweichend ist es auch möglich, einen der Ausgangsstoffe im Überschuß zu verwenden.

Üblicherweise setzt man Tricyclo [5.2.1.0^{2,6}] decan-8-on und das Amin der Formel R₁R₂NH im molaren Verhältnis 0,2:1 bis 1:1, insbesondere 0,7:1 bis 0,9:1 in die Reaktion ein.

Die Reste R₁ und R₂ des Amins der Formel R₁R₂NH können gleich oder verschieden sein. Sie stehen für einen Alkylrest mit 1 bis 6, insbesondere 1 bis 4, bevorzugt 1 bis 3 Kohlenstoffatomen.

Geeignete Amine sind Dimethylamin, Diethylamin, Di-n-propylamin, Di-n-butylamin, Di-i-butylamin, Di-n-pentylamin, Di-3-methylbutylamin, Di-n-hexylamin, Di-i-hexylamin, N-Methylethylamin, N-Methylpropylamin, N-Methylbutylamin, N-Methylpentylamin, N-Methylhexylamin, N-Ethylpropylamin, N-Ethylbutylamin, N-Ethylpentylamin, N-Ethylhexylamin, N-Propylbutylamin, N-Propylpentylamin, N-Propylhexylamin, N-Butylpentylamin, N-Butylhexylamin, insbesondere Dimethylamin, Diethylamin, Di-n-propylamin, bevorzugt Dimethylamin.

An den Platin enthaltenden Trägerkatalysator werden hinsichtlich des Platingehaltes keine besonderen Anforderungen gerichtet. Geeignet sind Katalysatoren, die 0,1 bis 10, insbesondere 0,2 bis 7, bevorzugt 0,5 bis 5 Gew.-% Platin enthalten. Es können auch Katalysatoren mit einem hohen Platingehalt verwendet werden.

Als Trägermaterial kommen Kieselgur, Kieselgel, SiO₂, Al₂O₃ oder Aktivkohle, insbesondere Al₂O₃ oder Aktivkohle, bevorzugt Aktivkohle in Betracht. Es können auch Gemische der vorstehend genannten Stoffe als Trägermaterial Anwendung finden.

Das erfindungsgemäße Verfahren erfordert einen Zusatz von Wasserstoff. Es empfiehlt sich Wasserstoff in Überschuß, bezogen auf den stöchiometrischen Bedarf, zu verwenden. Besonders einfach ist es, mittels Wasserstoff einen vorgegebenen Druck einzustellen und Wasserstoff in dem Maße, wie er durch die Umsetzung verbraucht wird, zu ergänzen. Obwohl die Reaktion bereits bei niedrigen Drücken einsetzt, empfiehlt es sich, bei Drücken von 0,5 bis 15, insbesondere 1,0 bis 10, bevorzugt 1,5 bis 8 MPa zu arbeiten.

Die Umsetzung beginnt schon bei Raumtemperatur abzulaufen. Zügiger läuft die Reaktion bei erhöhter Temperatur ab. Geeignete Temperaturen umfassen einen Bereich von 30 bis 150, insbesondere 40 bis 130, bevorzugt 50 bis 120°C.

Das erfindungsgemäße Verfahren läßt sich sowohl kontinuierlich als auch diskontinuierlich durchführen. Es eignet sich besonders für eine diskontinuierliche Arbeitsweise.

Die nachfolgenden Beispiele belegen die Erfindung, ohne sie zu beschränken.

### Experimenteller Teil

### Beispiel 1

### Herstellung von 8-N,N-Dimethylaminotricyclo [5.2.1.0^{2,6}] decan

In einem mit einem Rotationsrührer bestückten Autoklaven (Volumen 5 Liter) werden unter Ausschluß von Sauerstoff 2250 g (15 Mol) Tricyclo [5.2.1.0^{2,6}] decan-8-on und 11,25 g eines Katalysators, der 5 Gew.-% Platin auf Aktivkohle als Trägermaterial enthält, vorgelegt. 877 g (19,5 Mol) Dimethylamin werden in einen separaten Behälter gefüllt. Über eine Kapillare, die den separaten, mit Dimethylamin befüllten Behälter mit dem Autoklaven verbindet, wird das Dimethylamin mittels Wasserstoff in den Autoklaven gedrückt. Anschließend wird Wasserstoff bis zu einem Druck von 1,0 MPa aufgepreßt. Unter Rühren wird innerhalb von 40 Minuten auf die Reaktionstemperatur von 100°C aufgeheizt. Infolge der einsetzenden Reaktion sinkt der Druck auf 0,8 MPa ab. Durch Zugabe von Wasserstoff wird der Reaktionsdruck von 2,0 MPa eingestellt und durch wiederholte Wasserstoffzugabe aufrechterhalten.

Nach einer Stunde ist die Aufnahme von Wasserstoff beendet. Man läßt noch eine Stunde nachreagieren. Anschließend wird das Reaktionsprodukt abgekühlt und durch Druckfiltration vom Katalysator befreit.

Die Umsetzung führt zu folgenden Ergebnissen (jeweils bezogen auf eingesetztes Keton):
Umsatz: 96,2 % Selektivität: 98,5 % Ausbeute: 94,8 %

### Beispiel 2

In einem mit einem Rotationsrührer bestückten Autoklaven (Volumen 1 Liter) werden unter Ausschluß von Sauerstoff 300 g (2 Mol) Tricyclo [5.2.1.0^{2,6}] decan-8-on und 1,5 g des in Beispiel 1 beschriebenen Katalysators, der 5 Gew.-% Platin auf Aktivkohle als Trägermaterial enthält, vorgelegt. 117 g (2,6 Mol) Dimethylamin werden in einen separaten Behälter gefüllt. Über eine Kapillare, die den separaten, mit Dimethylamin befüllten Behälter mit dem Autoklaven verbindet, wird das Dimethylamin mittels Wasserstoff in den Autoklaven gedrückt. Danach wird, wie in Beispiel 1 angegeben, gearbeitet.

Nach 55 Minuten ist die Aufnahme von Wasserstoff beendet. Man läßt noch eine Stunde nachreagieren.

Anschließend wird das Reaktonsprodukt abgekühlt und durch Druckfiltration vom Katalysator befreit.

Die Umsetzung führt zu folgenden Ergebnissen (jeweils bezogen auf eingesetztes Keton):
Umsatz: 96,5 % Selektivität: 98,1 % Ausbeute: 94,7 %

### Beispiel 3

Es wird, wie in Beispiel 2 angegeben, gearbeitet, jedoch der in Beispiel 2 verwendete, durch Druckfiltration abgetrennte Katalysator eingesetzt (1. Wiedereinsatz des Katalysators).

Nach 55 Minuten ist die Aufnahme von Wasserstoff beendet. Man läßt noch eine Stunde nachreagieren.

Anschließend wird das Reaktionsprodukt abgekühlt und durch Druckfiltration vom Katalysator befreit.

Die Umsetzung führt zu folgenden Ergebnissen (jeweils bezogen auf eingesetztes Keton):
Umsatz: 96,5 % Selektivität: 98,2 % Ausbeute: 94,8 %

### Beispiel 4

Es wird, wie in Beispiel 2 angegeben, gearbeitet, jedoch der in Beispiel 3 verwendete, durch Druckfiltration abgetrennte Katalysator eingesetzt (2. Wiedereinsatz des Katalysators).

Nach 70 Minuten ist die Aufnahme von Wasserstoff beendet. Man läßt noch eine Stunde nachreagieren.

Anschließend wird das Reaktionsprodukt abgekühlt und durch Druckfiltration vom Katalysator befreit.

Die Umsetzung führt zu folgenden Ergebnissen (jeweils bezogen auf eingesetztes Keton):
Umsatz: 96,4 % Selektivität: 97,8 % Ausbeute: 94,3 %

Die Reaktionsbedingungen und die durch gaschromatografische Analyse ermittelte Zusammensetzung der Reaktionsprodukte sind der nachfolgenden Tabelle zu entnehmen.

**Tabelle**

| Beispiel | 1 | 2 | 3 | 4 |
|---|---|---|---|---|
| Temperatur [°C] | 100 | 100 | 100 | 100 |
| Druck [MPa] | 2,0 | 2,0 | 2,0 | 2,0 |
| Reaktionszeit [min] (einschl. Nachreaktion) | 120 | 115 | 115 | 130 |

| Einsatzstoffe | | | | |
|---|---|---|---|---|
| Keton¹⁾ [g] | 2250 | 300 | 300 | 300 |
| Dimethylamin [g] | 877 | 117 | 117 | 117 |
| Mol-Verhältnis Keton¹⁾:Dimethylamin | 0,77:1 | 0,77:1 | 0,77:1 | 0,77:1 |
| Katalysator (Gew.-% Pt bezogen auf Keton) | 0,025 | 0,025 | 0,025 | 0,025 |

| Zusammensetzung des Reaktionsproduktes (Gew.-%) | | | | |
|---|---|---|---|---|
| Vorlauf | 0,43 | 0,62 | 1,64 | 1,26 |
| Isomere | 0,45 | 0,54 | 0,27 | 0,55 |
| Dimethylamino tricyclodecan²⁾ | 92,62 | 92,23 | 91,47 | 91,03 |
| tertiäre Amine Keton¹⁾ | 0,35 | 0,32 | 0,45 | 0,45 |
| ungesättigtes Keton³⁾ | 3,61 | 3,38 | 3,34 | 3,39 |
| Alkohole⁴⁾ | 2,46 | 2,86 | 2,75 | 3,24 |
| Höhersieder | <0,08 | <0,05 | <0,08 | <0,08 |

| | | | | |
|---|---|---|---|---|
| ¹⁾ Tricyclo [5.2.1.0^{2,6}] decan-8-on | | | | |
| ²⁾ 8-N,N-Dimethylaminotricyclo [5.2.1.0^{2,6}] decan | | | | |
| ³⁾ Tricyclo [5.2.1.0^{2,6}] decan-8-on-3-en | | | | |
| ⁴⁾ 8-Hydroxytricyclo [5.2.1.0^{2,6}] decan + 8-Hydroxytricyclo [5.2.1.0^{2,6}] dec-3-en | | | | |

## Patentansprüche

1. Verfahren zur Herstellung von 8-N,N-Dialkylaminotricyclo [5.2.1.0^{2,6}] decan, dadurch gekennzeichnet, daß man Tricyclo [5.2.1.0^{2,6}] decan-8-on mit einem Amin der Formel R₁R₂NH, worin R₁ und R₂ für einen Alkylrest mit 1 bis 6 Kohlenstoffatomen stehen, in Gegenwart eines Platin enthaltenden Trägerkatalysators mit Wasserstoff bei erhöhtem Druck und erhöhter Temperatur umsetzt.

2. Verfahren nach Anspruch 1, dadurch gekennzeichnet, daß man Tricyclo [5.2.1.0^{2,6}] decan-8-on und das Amin der Formel R₁R₂NH im Verhältnis 0,2:1 bis 1:1, insbesondere 0,7:1 bis 0,9:1 einsetzt.

3. Verfahren nach Anspruch 1 oder 2, dadurch gekennzeichnet, daß im Amin der Formel R₁R₂NH R₁ und R₂ gleich oder verschieden sind.

4. Verfahren nach einem oder mehreren der Ansprüche 1 bis 3, dadurch gekennzeichnet, daß R₁ und R₂ für einen Alkylrest mit 1 bis 4, insbesondere 1 bis 3 Kohlenstoffatomen steht.

5. Verfahren nach einem oder mehreren der Ansprüche 1 bis 4, dadurch gekennzeichnet, daß als Amin Dimethylamin eingesetzt wird.

6. Verfahren nach einem oder mehreren der Ansprüche 1 bis 5, dadurch gekennzeichnet, daß der Platin enthaltende Trägerkatalysator 0,1 bis 10 insbesondere 0,2 bis 7, bevorzugt 0,5 bis 5 Gew.-% Platin enthält.

7. Verfahren nach einem oder mehreren der Ansprüche 1 bis 6, dadurch gekennzeichnet, daß der Platin enthaltende Trägerkatalysator Al₂O₃, Kieselgur, Kieselgel, SiO₂, Aktivkohle, insbesondere Al₂O₃ oder Aktivkohle, bevorzugt Aktivkohle als Trägermaterial enthält.

8. Verfahren nach einem oder mehreren der Ansprüche 1 bis 7, dadurch gekennzeichnet, daß man die Umsetzung bei 0,5 bis 15, insbesondere 1,0 bis 10, bevorzugt 1,5 bis 8 MPa durchführt.

9. Verfahren nach einem oder mehreren der Ansprüche 1 bis 8, dadurch gekennzeichnet, daß man die Umsetzung bei 30 bis 150, insbesondere 40 bis 130, bevorzugt 50 bis 120 °C durchführt.

## Claims

1. A process for preparing 8-N,N-dialkylaminotricyclo[5.2.1.0^{2,6}]decane, which comprises tricyclo[5.2.1.0^{2,6}]decan-8-one with an amine of the formula R₁R₂NH, where R₁ and R₂ are an alkyl radical having 1 to 6 carbon atoms, in the presence of a platinum-containing supported catalyst with hydrogen at elevated pressure and elevated temperature.

2. The process as claimed in claim 1, wherein tricyclo[5.2.1.0^{2,6}]decan-8-one and the amine of the formula R₁R₂NH are used in the ratio 0.2:1 to 1:1, particularly 0.7:1 to 0.9:1.

3. The process as claimed in claim 1 or 2, wherein R₁ and R₂ in the amine of the formula R₁R₂NH are identical or different.

4. The process as claimed in one or more of claims 1 to 3, wherein R₁ and R₂ are an alkyl radical having 1 to 4, in particular 1 to 3 carbon atoms.

5. The process as claimed in one or more of claims 1 to 4, wherein the amine used is dimethylamine.

6. The process as claimed in one or more of claims 1 to 5, wherein the platinum-containing supported catalyst contains 0.1 to 10, particularly 0.2 to 7, and preferably 0.5 to 5% by weight of platinum.

7. The process as claimed in one or more of claims 1 to 6, wherein the platinum-containing supported catalyst contains Al₂O₃, kieselguhr, silica gel, SiO₂, activated charcoal, in particular Al₂O₃ or activated charcoal and preferably activated charcoal, as support.

8. The process as claimed in one or more of claims 1 to 7, wherein the reaction is carried out at a pressure of 0.5 to 15, particularly 1.0 to 10, and preferably 1.5 to 8 MPa.

9. The process as claimed in one or more of claims 1 to 8, wherein the reaction is carried out at 30 to 150, particularly 40 to 130, and preferably 50 to 120°C.

## Revendications

1. Procédé de préparation de 8-N,N-dialkylaminotricyclo[5.2.1.0^{2,6}]décanes, caractérisé en ce que l'on fait réagir la tricyclo[5.2.1.0^{2,6}]décane-8-one et une amine de formule R₁R₂NH dans laquelle R₁ et R₂ représentent des groupes alkyles en C₁-C₆, en présence d'un catalyseur au platine sur support, avec de l'hydrogène à pression et température élevées.

2. Procédé selon revendication 1, caractérisé en ce que l'on met en oeuvre la tricyclo[5.2.1.0^{2,6}]décane-8-one et l'amine de formule R₁R₂NH dans des proportions relatives de 0,2 : 1 à 1 : 1 et plus spécialement de 0,7 : 1 à 0,9 : 1.

3. Procédé selon la revendication 1 ou 2, caractérisé en ce que, dans la formule de l'amine R₁R₂NH, R₁ et R₂ ont des significations identiques ou différentes.

4. Procédé selon une ou plusieurs des revendications 1 à 3, caractérisé en ce que R₁ et R₂ représentent des groupes alkyles en C₁-C₄, plus spécialement en C₁-C₃.

5. Procédé selon une ou plusieurs des revendications 1 à 4, caractérisé en ce que l'amine mise en oeuvre est la diméthylamine.

6. Procédé selon une ou plusieurs des revendications 1 à 5, caractérisé en ce que le catalyseur au platine sur support contient de 1,1 à 10, plus spécialement de 0,2 à 7 et de préférence de 0,5 à 5 % en poids de platine.

7. Procédé selon une ou plusieurs des revendications 1 à 6, caractérisé en ce que le catalyseur au platine sur support contient en tant que matière de support Al₂O₃, du kieselgur, du gel de silice, SiO₂, du charbon actif, plus spécialement Al₂O₃ ou du charbon actif, et de préférence du charbon actif.

8. Procédé selon une ou plusieurs des revendications 1 à 7, caractérisé en ce que la réaction est effectuée à des pressions de 0,5 à 15, plus spécialement de 1,0 à 10 et de préférence de 1,5 à 8 MPa.

9. Procédé selon une ou plusieurs des revendications 1 à 8, caractérisé en ce que la réaction est effectuée à des températures de 30 à 150, plus spécialement de 40 à 130 et de préférence de 50 à 120°C.
